# EUROPEAN PATENT SPECIFICATION

(11) **EP 2 121 123 B1**
(45) Date of publication and mention of the grant of the patent: **22.08.2012**
(21) Application number: 07862119.0
(22) Date of filing: 19.11.2007
(51) Int. Cl.: A61N 1/32, A61N 1/36, A61B 5/20

(54) **RENAL FUNCTION MODULATION VIA ELECTRICAL ENERGY STIMULATION**
MODULIERUNG DER NIERENFUNKTION DURCH STIMULIERUNG MIT ELEKTRISCHER ENERGIE
MODULATION DE LA FONCTION RENALE PAR STIMULATION D'ENERGIE ELECTRIQUE

(30) Priority: 22.11.2006 US 562436
(43) Date of publication of application: 25.11.2009
(73) Proprietor: Cardiac Pacemakers, Inc., St. Paul, MN 55112-5798 (US)
(72) Inventor: STAHMANN, Jeffrey E., Ramsey, MN 55303 (US)
(74) Representative: Pfenning, Meinig & Partner GbR
(86) International application number: PCT/US2007/024185
(87) International publication number: WO 2008/066732

(56) References cited:
- WO-A-00/74775
- WO-A-01/52931
- WO-A-2006/090397
- WO-A-2007/019491
- US-A- 5 529 574
- US-A1- 2003 216 792
- US-A1- 2004 220 621
- US-A1- 2005 021 092
- US-A1- 2006 041 277
- US-A1- 2006 206 150
- US-A1- 2007 083 239

## Description

### TECHNICAL FIELD

This patent document pertains generally to medical systems and methods. More specifically, this patent document pertains to renal function modulation via application of electrical energy stimulation.

### BACKGROUND

Kidneys are vital organs that perform many functions including regulation of water and electrolytes, excretion of metabolic wastes and bioactive substances, and regulation of arterial blood pressure, red blood cell production and vitamin D. Every day, the kidneys process about 200 quarts of blood to sift out about 2 quarts of waste products and water. The waste and extra water become urine, which flows to one's bladder through tubes called urethers. The bladder stores the urine until it is excreted. The wastes in the blood come from the normal breakdown of active bodily tissues and from consumed food. The body uses the food for energy and self-repairs. After the body has taken what it needs from the food, waste is sent to the blood. If the kidneys do not remove this waste, the waste builds-up in the blood and may damage the body.

The actual filtering in the kidneys occurs via tiny units therein called nephrons. In each nephron, a group of interconnected capillary loops, called the glomerulus, filters the blood and produces a fluid, called the filtrate. The filtrate is similar to blood plasma but contains very little total protein. Unlike large proteins (e.g. albumin), inorganic ions and low-molecular-weight organic solutes are freely filtered by the glomerulus into the filtrate. Since the inorganic ions and low-molecular-weight organic solutes are freely filtered, their concentrations in the filtrate are very similar to their concentration in blood plasma. The filtrate leaving the glomerulus contains a combination of waste materials that need to be removed from the body, other solutes (e.g. electrolytes) - some of which need to be removed from the body and some of which need to be retained by the body, and water - most of which needs to be retained by the body. To affect the removal and retention of these substances, the filtrate leaving the glomerulus empties into a tiny tube called a tubule.

Several processes, including reabsorption and secretion, occur within the tubule. These processes, combined with filtration by the glomerulus, affect proper retention and removal of the various solutes and water. Most of the water and other solutes (e.g. glucose, electrolytes, bicarbonate) are reabsorbed as the filtrate moves though the tubule. The process of reabsorption is critical since without it, the body would quickly dehydrate and suffer electrolyte and pH imbalances. Secretion occurs within the tubule and is critical for many processes, for example, pH balance (hydrogen ion secretion) and potassium balance. Some of the water and solutes (e.g. urea) pass through the tubule, thus producing urine. In addition to the secreted substances described above, the kidneys release important hormones, such as erythropoietin (EPO), which stimulates bone marrow to make red blood cells; renin, which regulates blood pressure; and calcitriol, which helps maintain calcium for bones and for normal chemical balance in the body. Still other functions performed by the kidneys include maintenance of the body's control of several important endocrine functions.

Unfortunately, a number of people experience progressively worsening renal failure as a result of a variety of disorders. As one or more of the disorders worsen, a person typically cannot live long without some form of renal (i.e., kidney) therapy. In many instances, the treatment of renal failure attempts to address secondary symptoms of the failure, rather than directly impact the function of the kidneys themselves. For example, diuretics are often given to reduce blood volume and pain medication is often given to alleviate subject discomfort. As another example, end stage renal failure is typically treated by hemodialysis (where the blood is artificially "cleaned" by exchange with a dialysis fluid across a selectively permeable membrane) or by transplantation, both of which have numerous associated drawbacks. Dialysis subjects, for instance, must adhere to rigid dialysis schedules that are typically on the order of four hours at a time, three times per week. Dialysis subjects must also restrict fluid intake, follow strictly controlled diets, take daily medications, and endure such things as anemia, abnormal bone metabolism, chronic uremia, and diminished sexual function. An alternative to hemodialysis is transplantation. However, transplantation also has associated drawbacks, including being an inherently risky procedure and the risk of organ rejection. Additionally, transplantation is at the mercy of organ supply, which currently is experiencing growing shortages.

Document US 5 529 574 A refers to a catheter for providing low level electrical stimulation to a prostate gland to relieve the deleterious effects of prostatic disorders that respond to low level electrical stimulation. The catheter is provided in combination with a power supply adapted to provide low voltage direct current or low voltage, low frequency alternating current of less than about 30KHz.

Document US 2003/216792 A1 refers to methods and apparatus for treatment of congestive heart failure, chronic renal failure and hypertension by nerve stimulation. In particular, the document relates to the improvement of these conditions of patients by blocking signals to the renal nerve.

Document WO 2006/090397 A refers to an adaptive feed-back controlled system for regulating a physiological function of a heart in which a hemodynamic sensor continuously monitors the physiological performance of the heart.

Document WO 01/52931 A discusses a blood flow controller for treating a tissue condition by electrifying a selected blood vessel with an electric field that modifies blood flow associated with a treated tissue by directly acting on the blood vessel, receiving an automatic indication of a local effect of electrification, and varying the electrification responsive to the indication.

### SUMMARY

Given the wide range of important functions that the kidneys provide, it is desirable to maintain the kidneys in a state of relative well-being, including modulating kidney function prior to, during, or following renal disease or other degenerative disorders.

The invention is defined by claim 1 and includes a system for applying a stimulus to at least one of a glomerulus, a Bowman's capsule, a macula densa, a tubule, a peritubular capillary network, a collecting duct, an afferent arteriole, an efferent arteriole, or a renal granular cell within a kidney of a subject. The system includes, among other things, a first electrode, a second electrode, and an electrical energy delivery circuit. The electrical energy delivery circuit is coupled to the first electrode and the second electrode to deliver a generated first electrical energy signal having a frequency between about 1KHz and about 1MHz. The first electrode and the second electrode are positioned and configured to direct a substantially large portion of the first electrical signal through at least one of the glomerulus, the Bowman's capsule, the macula densa, the tubule, the peritubular capillary network, the collecting duct, the afferent arteriole, the efferent arteriole, or the renal granular cell to modulate one or more renal functions.

Advantageously, the present subject matter may keep kidney subjects in a state of relative well-being by preventing, delaying, or minimizing renal conditions including, for example, chronic kidney disease and end stage renal failure via application of internal electrical energy stimulation. The electrical energy stimulation may be used conjunctively or in lieu of drug or other therapies to modulate one or more renal functions. In this way, the electrical energy stimulation provides an option for subjects who respond inadequately to drug therapy, are intolerant of drug therapy, have preference for treatment via electrical energy stimulation, or are non-compliant with drug therapy and may further modulate renal functions that are beyond the reach of existing drug therapy. Yet another advantage of the present subject matter is that it can be configured such that subject action or compliance is not needed for resulting improvement of subject health.

This Summary is an overview of some of the teachings of the present patent document and not intended to be exclusive or exhaustive treatment of the present subject matter. Further details about the present subject matter are found in the detailed description and appended claims. Other aspects and advantages will be apparent to persons skilled in the art upon reading and understanding the following detailed description and viewing the drawings that form a part thereof, each of which are not to be taken in a limiting sense. The scope of the present subject matter is defined by the appended claims and their legal equivalents.

### BRIEF DESCRIPTION OF THE DRAWINGS

In the drawings, like numerals describe substantially similar components throughout the several views. The drawings illustrate generally, by way of example, various embodiments discussed in the present document.
- FIG. **1**: is a schematic view of a system for delivering electrical energy stimulation to one or more portions of a subject's body, including a subject's kidney(s), according to one embodiment of the present subject matter.
- FIG. **2**: is a block diagram of a system for delivering electrical energy stimulation to one or more portions of a subject's body, including a subject's left kidney, according to one embodiment of the present subject matter.
- FIG. **3A**: is a schematic view of a system in the course of delivering electrical energy stimulation in the form of an electric current or an electrical field to a portion of a subject's left kidney, according to one embodiment of the present subject matter.
- FIG. **3B**: diagrammatically illustrates a nephron of a kidney to which electrical energy stimulation can be delivered, according to one embodiment of the present subject matter.
- FIG. **4A**: is a schematic view of kidney structures associated with one or more renal functions that may be modulated via application of electrical energy stimulation, according to one embodiment of the present subject matter.
- FIG. **4B**: is an enlarged view of one or more kidney structures alterable via application of electrical energy stimulation, according to one embodiment of the present subject matter.
- FIG. **4C**: is an enlarged view of various kidney structure transport mechanisms alterable via application of electrical energy stimulation, according to one embodiment of the present subject matter.
- FIG. **5**: illustrates a method of modulating one or more renal functions using electrical energy stimulation, according to one embodiment of the present subject matter.

### DETAILED DESCRIPTION

The following detailed description of the present subject matter refers to subject matter in the accompanying drawings which show, by way of illustration, specific embodiments in which the present subject matter may be practiced. References to "an", "one", or "various" embodiments in this patent document are not necessarily to the same embodiment, and such references contemplate more than one embodiment. The following detailed description is demonstrative and not to be taken in a limiting sense. The scope of the present subject matter is defined by the appended claims, along with the full scope of legal equivalents to which such claims are entitled.

Various embodiments of the present subject matter are provided herein for renal function modulation via application of electrical energy stimulation. The electrical energy stimulation can be used to supplement or in lieu of existing treatments affecting renal function (e.g., drug therapy, hemodialysis or transplantation, among others) to keep kidney subjects in a state of relative well-being by preventing, delaying, or minimizing renal conditions including, for example, chronic kidney disease and end stage renal failure. It is believed that by selectively manipulating (via application of electrical energy stimulation) one or more kidney structures (e.g., a glomerulus, a Bowman's capsule, a macula densa, a tubule, a peritubular capillary network, a collecting duct, an afferent arteriole, an efferent arteriole, or a renal granular cell) one or more renal functions performed by such structures may be modulated allowing a desired biological response of one or more renal function-associated parameters (e.g., an electrolyte level, a water level, a metabolic waste level (including a creatinine level, a blood urea nitrogen level, or a uric acid level), a pharmacological agent level, a hormone level, a blood pressure level, an erythropoietin level, a vitamin D level, a glucose level, a pH level, or a glomerulus filtration rate level) to be effectuated. By altering the one or more renal function-associated parameters as desired, it is further believed that associated diseases (e.g., hypertension, edema, heart failure, blood electrolyte imbalances, and others) may be treated or prevented.

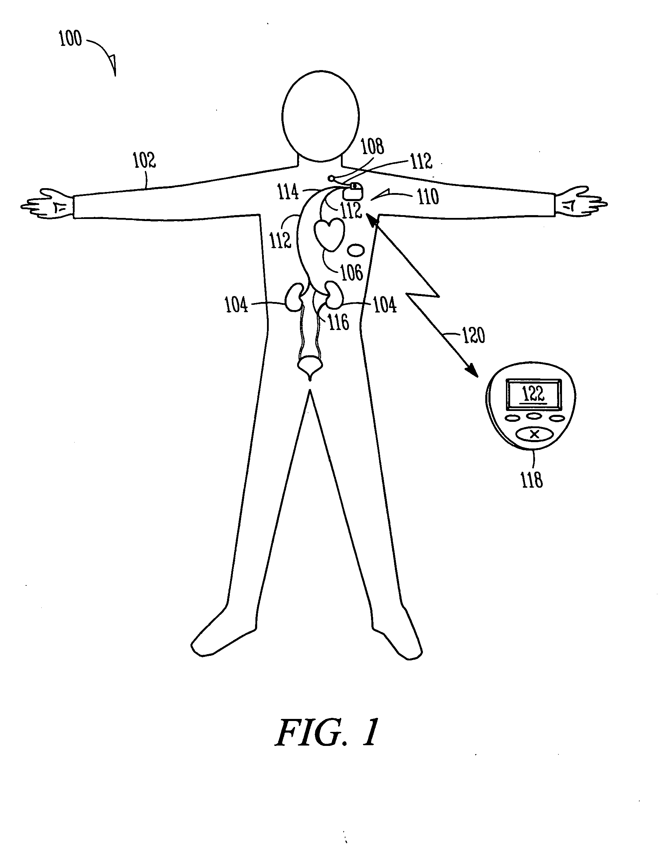

FIG. **1** schematically illustrates one embodiment of a system **100** for delivering electrical energy stimulation to one or more portions of a subject's body **102**, such as one or both kidneys **104**, the heart **106**, or an efferent parasympathetic nerve **108**. While not shown, the system **100** can also be configured to deliver electrical energy stimulation to other portions of the subject's body **102**, such as the brain or pulmonary regions. In this embodiment, the system **100** includes an implantable medical device (IMD) **110**, such as a pulse generator including cardiac therapy capabilities (e.g., capable of providing one or more of bradycardia therapy, tachycardia therapy, or cardiac resynchronization therapy), which is coupled by one or more leads **112** to the kidneys **104**, the heart **106**, and the efferent para-sympathetic nerve **108**. The IMD **110** can be implanted subcutaneously in the subject's chest, abdomen, or elsewhere. Each of the one or more leads **112** extends from a lead proximal end portion **114** to a lead distal end portion **116**, the latter of which includes one or more electrodes for delivering the electrical energy stimulation generated by the IMD **110** to the kidney(s) **104**, the heart **106,** or the efferent parasympathetic nerve **108**.

The exemplary system **100** shown also includes an external user-interface **118**. The external user-interface **118** can be used to receive information from, or send information to, the IMD **110**. For instance, new values for one or more electrical energy parameters (e.g., an energy injection location, an energy injection duration, an energy injection intensity, an energy injection frequency, an energy injection polarity, an energy injection electrode configuration, or an energy injection waveform) applied to one or more kidney structures (e.g., a glomerulus, a Bowman's capsule, a macula densa, a tubule, a peritubular capillary network, a collecting duct, an afferent arteriole, an efferent arteriole, or a renal granular cell) can be manually input into the external user-interface **118** and sent to the IMD **110** so-as-to change a parameter of the electrical energy stimulation resulting in a desired biological response of one or more renal function-associated parameters (e.g., an electrolyte level, a water level, a metabolic waste level (including a creatinine level, a blood urea nitrogen level, or a uric acid level), a pharmacological agent level, a hormone level, a blood pressure level, an erythropoietin level, a vitamin D level, a glucose level, a pH level, or a glomerulus filtration rate level). Additionally, the external user-interface **118** can be used to receive one or more inputs of the subject's **102** health-related information. In certain embodiments, the external user-interface **118** is used to externally process information for the system **100**. Using telemetry or other known communication techniques, the external user-interface **118** can wirelessly communicate **120** with the IMD **110**. As shown, the external user-interface **118** can include a visual or other display unit **122**, such as an LCD or LED display, for textually or graphically relaying information to the subject **102** or a caregiver regarding operation or findings of the system **100**.

While the present system **100** is useful in sensing and/or stimulating many portions of a subject's **102** body, particular attention will hereinafter be made to the present system's **100** use with one or more portions of a subject's kidney(s), and more specifically, with one or more of the glomerulus, the Bowman's capsule, the macula densa, the tubule, the peritubular capillary network, the collecting duct, the afferent arteriole, the efferent arteriole, or the renal granular cell.

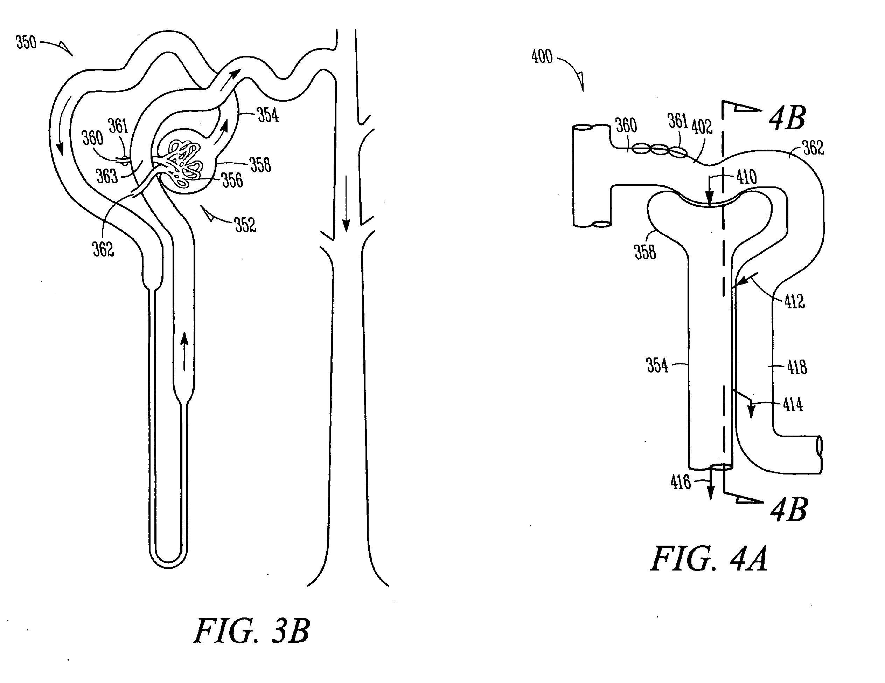

As discussed above, the actual filtering in the kidneys **104** occurs via tiny units therein called nephrons **350** (FIG. **3B**). Each kidney has about a million nephrons **350**. It is known that the major cause of renal failure is not a change in the filtration properties of working nephrons but rather a decrease in the number of functioning nephrons **350**. As some nephrons **350** become diseased, others compensate by enlarging and assuming a portion of the lost function. Over time, more and more of the nephrons **350** become diseased to the point where the working nephrons **350** are unable to provide, among other things, the needed filtration, electrolyte balance, or hormonal balance to the kidney **104** for adequate performance thereof. Such inadequate kidney **104** performance is likely to result in disease-indicative levels of one or more renal function-associated parameters (e.g., an electrolyte level, a water level, a metabolic waste level (including a creatinine level, a blood urea nitrogen level, or a uric acid level), a pharmacological agent level, a hormone level, a blood pressure level, an erythropoietin level, a vitamin D level, a glucose level, a pH level, or a glomerulus filtration rate level).

To restore kidney performance, the present subject matter is provided. It is believed that by artificially stimulating (via the application of electrical energy stimulation) those nephrons **350** and/or associated renal structures, that for various reasons have stopped contributing, or contribute in a reduced fashion, to the overall functions of the kidney **104**, renal performance may be affected in a positive way. Additionally, it is believed that electrical energy stimulation of nephrons **350** and/or associated renal structures will provoke normally functioning nephrons **350** and/or associated renal structures into a state of hyperfunctionality thus compensating for renal function lost due to malfunctioning nephrons **350** and/or malfunctioning renal functions associated with such nephrons **350**. In various embodiments, the electrical energy stimulation is applied to one or more renal structures (e.g., a glomerulus, a Bowman's capsule, a macula densa, a tubule, a peritubular capillary network, a collecting duct, an afferent arteriole, an efferent arteriole, or a renal granular cell) thereby modulating one or more renal functions. It is further believed that the modulation of the one or more renal functions, in turn, results in nondisease-indicative levels and/or reduced disease-indicative levels of the one or more renal function-associated parameters.

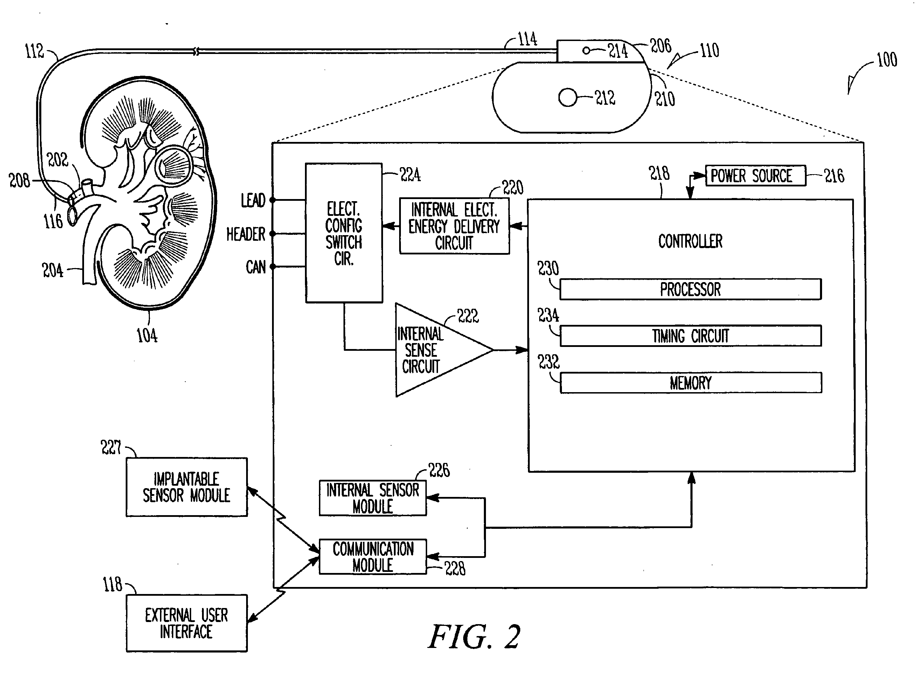

The simplified block diagram of FIG. **2** illustrates one conceptual embodiment of the system **100**, which can deliver the electrical energy stimulation to the subject's **102** (FIG. 1) kidney(s) **104**. As shown, the system **100** includes an IMD **110**, such as a pulse generator, coupled via one or more leads **112** to a kidney **104**, such as the left kidney **104**. In this embodiment, the one or more leads **112** are providing vascular access to the kidney **104** via a renal vein **202.** In another embodiment, the one or more leads **112** can be provided access to the kidney **104** via a ureter **204** access.

Each lead **112** extends from a lead proximal end portion **114**, which is coupled to an insulating header **206** of the IMD **110**, to a lead distal end portion **116**, positioned within the renal region. Each lead distal end portion **116** includes one or more electrodes **208** for delivering the electrical energy stimulation generated by the IMD **110**. The one or more electrodes **208** can also be used for sensing information about one or more renal function-associated parameters, which can then be used by the IMD **110** (e.g., a processor **230**) to calculate a kidney status indicative signal, which indicates at least one of the absence, presence, increase, decrease, occurrence, termination, impending change, or rate of change of one or more renal functions. The kidney status indicative signal can, in turn, be used for proper electrical energy stimulation generation and delivery (e.g., an energy injection location, an energy injection duration, an energy injection intensity, and energy injection frequency, an energy injection polarity, an energy injection electrode configuration, or an energy injection waveform). In addition to the lead electrodes **208**, other electrodes usable in the delivery of the electrical energy stimulation can be located on a hermetically-sealed enclosure **210** of the IMD **110** (typically referred to as a can electrode **212)** or on the insulating header **206** (typically referred to as a header electrode **214)**.

As shown, the IMD **110** includes electronic circuitry components that are enclosed within the hermetically-sealed enclosure **210**, such as a controller **218**, a power source **216**, an electrical energy delivery circuit **220**, an internal sense circuit **222**, an electronic configuration switch circuit **224**, an internal sensor module **226**, and a communication module **228**. The power source **216** provides operating power to all of the aforementioned IMD internal modules and circuits. In certain embodiments, the power source **216** should be capable of operating at low current drains for long periods of times.

The controller **218** includes, among other things, a processor **230**, a memory **232**, and a timing circuit **234**. The processor **230** is configured to determine an electrical energy signal command using information about a desired biological response of one or more renal function-associated parameters. The electrical energy signal command is subsequently communicated to the electrical energy delivery circuit **220**, which is configured to generate an electrical energy signal deliverable by one or more chosen electrodes **208**, **212**, or **214** to the kidney **104**. In various examples, the one or more delivery electrodes are chosen such that a substantially large portion of the electrical energy signal passes through one or more kidney structures (e.g., a glomerulus, a Bowman's capsule, a macula densa, a tubule, a peritubular capillary network, a collecting duct, an afferent arteriole, an efferent arteriole, or a renal granular cell). The electrical energy circuit **220** is selectively coupled to the one or more electrodes **208**, **212**, or **214** by the electronic configuration switch circuit **224**.

The electrical energy stimulation can be delivered to the kidney **104** in various ways. For instance, the electrical energy stimulation delivered to the kidney **104** by the electrodes **208**, **212**, or **214** includes a frequency equal to or greater than about 1KHz. In one such embodiment, the signal frequency equal to or greater than about 1KHz is delivered in one or more bursts having a burst frequency substantially less than 1KHz, such as around 1Hz. In another embodiment, the electrical energy stimulation delivered to the kidney **104** by the electrodes **208**, **212**, or **214** includes a frequency of greater than about 50KHz. In yet another embodiment, the electrical energy stimulation delivered to the kidney **104** by the electrodes **208**, **212**, or **214** includes a continuous periodic or pulsed periodic electric current or voltage. In still other embodiments, the electrical energy stimulation can include a frequency substantially below 1KHz.

The internal sense circuit **222** and the internal sensor module **226** (i.e., one or more measurement units) are configured to sense information about then-current values of the one or more renal function-associated parameters. From the internal sense circuit **222** and the internal sensor module **226**, the parameter information is sent to the controller **218** for processing (e.g., calculation of a kidney status indicative signal) by the processor **230**. The processor **230** can compare the then-current values of the one or more renal function-associated parameters (or then-current kidney indicative signal) to the desired parameter values (or desired kidney indicative signal) stored in the memory **232** and thereafter determine whether the electrical energy stimulation command communicated to the energy delivery circuit **220** needs to be adjusted or terminated.

The system **100** of this embodiment further includes an external user-interface **118** and an implantable sensor module **227** (i.e., measurement units or display devices not physically connected to the IMD **110**). The external user-interface **118** receives, for example, manually entered desired values of the one or more renal function-associated parameters and communicates the same to the IMD **110** via the communication module **228**. The manually entered values can be used in lieu of preprogrammed parameter values stored in the memory **232**. The implantable sensor module **227** includes sensors to measure information about then-current values of the one or more parameters and relays such information to the IMD **110** via the communication module **228**.

It is to be noted that FIG. **2** illustrates just one conceptualization of various modules, circuits, and interfaces of system **100**, which are implemented either in hardware or as one or more sequences of steps carried out on a microprocessor or other controller. Such modules, circuits, and interfaces are illustrated separately for conceptual clarity; however, it is to be understood that the various modules, circuits, and interfaces of FIG. **2** need not be separately embodied, but may be combined or otherwise implemented.

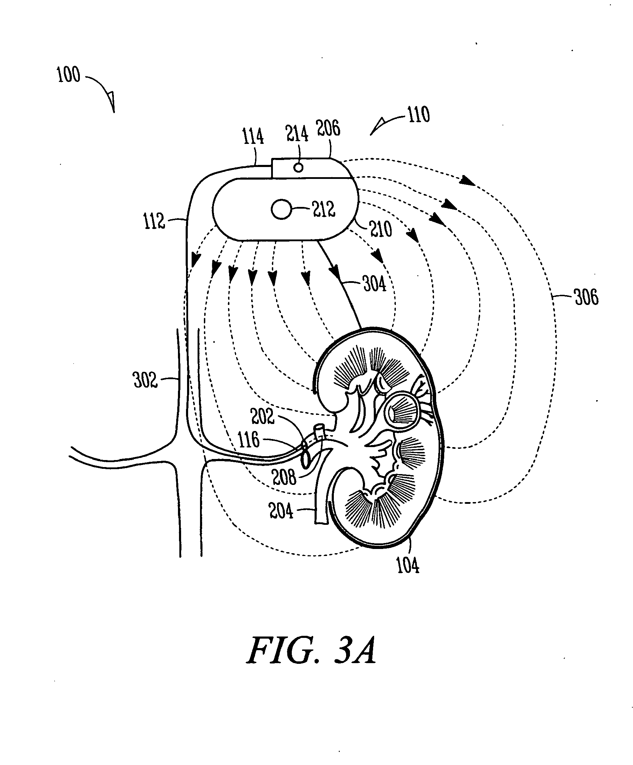

FIG. **3A** schematically illustrates the system **100** in the process of delivering electrical energy stimulation in the form of an electric current **304** and an associated electric field **306** to the subject's kidney **104.** In certain embodiments, the electrical energy stimulation includes a pulsed voltage signal with approximately a zero average amplitude, a frequency between approximately 1KHz and approximately 1MHz, and a peak-to-peak amplitude sufficient to produce an electric field strength of approximately 10 volts per centimeter. As shown, the kidney **104** is a bean-shaped structure, the rounded outer convex of which faces the side of the subject's body **102** (FIG. 1). The inner, indented surface of the kidney **104**, called the hilum, is penetrated by a renal artery, a renal vein **202**, nerves, and a ureter **204**, the latter of which carries urine out of the kidney **104** to the bladder (*see* FIG. 1). As shown, the system **100** includes an IMD **110** electrically coupled to the kidney **104** via at least one lead **112**. The lead extends from a lead proximal end portion **114**, where it is coupled to an insulated header **206** of the IMD **110**, to a lead distal end portion **116** disposed within the renal vein **202**. In this embodiment, the lead **112** is provided vascular access to the renal vein **202** via the inferior vena cave **302**. In another embodiment, the lead distal end portion **116** is positioned deep within the kidney **104**, such as in an arcuate vein, an interlobar vein, or a segmental vein. In yet another embodiment, the lead **112** can be delivered via a urethrabladder-ureter **204** access.

As shown, but as may vary, the lead distal end portion **116** includes at least one implanted electrode **208** disposed proximal to the kidney **104** (i.e., within, on, or about the kidney **104**), while the hermetically-sealed enclosure **210** (via can electrode **212**) or the insulating header **206** (via header electrode **214**) acts as another implanted electrode by being at least partially conductive. In this way,
an electrical energy signal provided by the IMD **110** and delivered by the lead electrode **208** disposed within, on, or about the kidney **104** can return through a portion of the kidney to the can **212** or header **214** electrode. In certain embodiments, the electrical energy stimulation is delivered in the form of an electric current **304** having an associated electric field **306**.

The electric current **304** and the associated electric field **306** can be positioned such that one or more structures of the kidney **104** (e.g., a glomerulus, a Bowman's capsule, a macula densa, a tubule, a peritubular capillary network, a collecting duct, an afferent arteriole, an efferent arteriole, or a renal granular cell) are immersed within the current **304** or field **306** sufficient to affect one or more renal functions, and more specifically, affect one or more parameters associated with the one or more renal functions (e.g., an electrolyte level, a water level, a metabolic waste level (including a creatinine level, a blood urea nitrogen level, or a uric acid level), a pharmacological agent level, a hormone level, a blood pressure level, an erythropoietin level, a vitamin D level, a glucose level, a pH level, or a glomerulus filtration rate level). The present system **100** is adapted to work in a variety of electrode configurations and with a variety of electrical contacts (e.g., patches) or electrodes in addition to the electrode configuration shown in FIG. **3A**. For instance, multiple leads **112** can be placed in different kidney locations to improve the electric current **304** or electric field **306** distributions. Alternatively or additionally, lead **112** can have one or more additional electrodes wherein the one or more electrodes perform as the cathode for the electric current **304** and associated electric field **306**, for example.

FIG. **3B** diagrammatically illustrates one of many nephrons **350** in a kidney **104** (FIG. **1**). As discussed above, the nephrons **350** perform the actual filtering in the kidneys **104**. It follows that in order to modulate one or more functions of the kidney **104**, the function of one or more nephrons **350** or their associated structures need to be modulated. For better understanding of how the present subject matter may be used to affect one or more renal functions, discussion will now turn to the modulation of a nephron **350** and its associated structure.

Each nephron consists of a spherical filtering component, called the renal corpuscle **352**, and a tubule **354** extending from the renal corpuscle **352**. The renal corpuscle **352** is responsible for the initial step in urine formation (i.e., the separation of a protein-free filtrate from plasma) and consists of interconnected capillary loops (the glomerulus **356**) surrounded by a hollow capsule (Bowman's capsule **358**). Blood enters and leaves Bowman's capsule **358** through afferent and efferent arterioles **360**, **362** that penetrate the surface of the capsule **358.** Proximal to the arterioles **360**, **362** are one or more renal granular cells **361**, the latter of which stimulate the release of renin upon change in systemic blood pressure. A fluid-filled space exists within the capsule **358**, and it is into this space that fluid filters. Opposite the vascular pole, Bowman's capsule **358** has an opening that leads into the first portion of the tubule **354**. Specialized cells in the thick ascending limb of the tubule **354** closest to the Bowman's capsule **358** constitute the macula densa **363**, which generates signals that influence the rennin-angiotensin system. The filtration barrier in the renal corpuscle **352** through which all filtered substances pass consists of three layers: the capillary endothelium of the glomerular capillaries, a basement membrane, and a single-celled layer of epithelial cells.

FIG. **4A** illustrates portions of the renal process **400**, which includes glomerular filtration **410**, tubular secretion **412**, tubular reabsorption **414**, and excretion **416.** Urine formation begins with glomerular filtration **410**, which includes the bulk flow of fluid from the glomerular capillaries **402** into Bowman's capsule **358**. Many low-molecular weight components of blood are freely filtered during glomerular filtration **410**. Among the most common substances included in the freely filtered category are the ions sodium, potassium, chloride, and bicarbonate; the neutral organics glucose and urea; amino acids; and peptides like insulin and antidiuretic hormone (ADH).

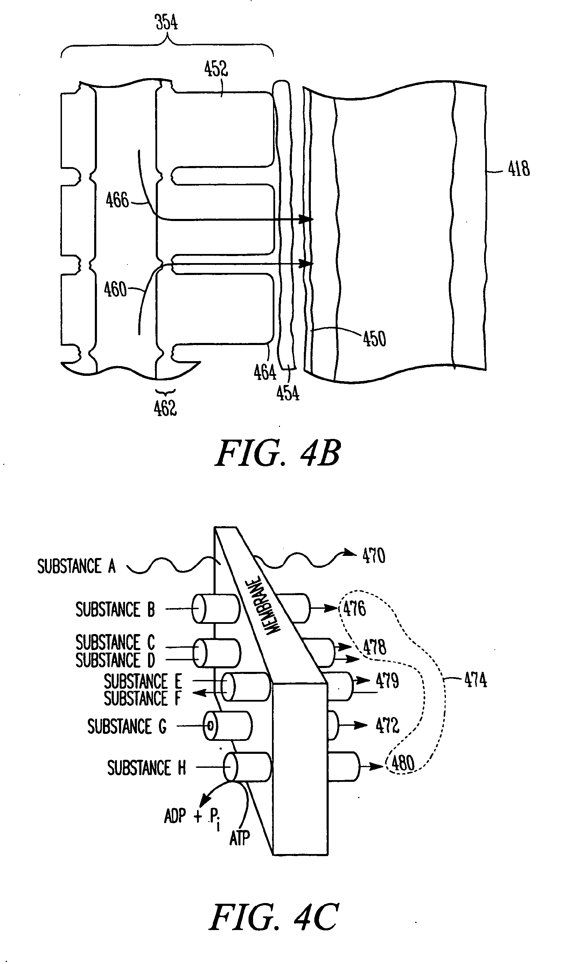

As the filtrate flows from Bowman's capsule **358** through the various portions of the tubule **354**, its composition is altered, mostly by removing material (tubular reabsorption **414**) but also by adding material (tubular secretion **412**). The tubule **354** is, at all points, intimately associated with peritubular capillaries **418**, a relationship that permits the transfer of materials between the capillary plasma and the lumen of the tubule **354**. As shown in FIG. **4B**, the basic processes of tubular reabsorption **414** and tubular secretion **412** involve crossing two barriers: the tubular epithelium **452** and the endothelial cells **450** lining the peritubular capillaries **418**.

For reabsorbed substances, the endothelial cell barrier **450** is like the barrier of many other peripheral capillary beds in the body - solutes cross the peritubular capillary barrier through the basement membrane **454** and then the fenestrae in the endothelial cells **450**. For secreted substances, crossing the endothelium **450** is similar to the filtration process in the glomerular capillaries **402** (FIG. **4A**), but it is traveling in the opposite direction. However, because the endothelium **450** is highly permeable to small solutes, this is quite feasible providing there is a suitable concentration gradient.

Crossing the epithelium **452** lining the tubule **354** can be performed in a single step or in two steps. The paracellular route **460** (single step) is when the substance goes around the cells (i.e., through the matrix of the tight junctions that link each epithelial cell **452** to its neighbor). More typically, however, the substances travel through the cells in a two-step process - across the apical membrane **462** facing the tubular lumen and across the basolateral membrane **464** facing the interstitium. This is called the transcellular route **466**.

Arrays of mechanisms exist by which substances cross the various barriers. Renal cells use whichever set of tools is most suitable for the task. The general classes of mechanisms for traversing the barriers are illustrated in FIG. **4C** and include movement by diffusion **470**, movement through channels **472**, and movement by transporters **474**.

Diffusion **470** is the random movement of free molecules in a solution. Net diffusion **470** occurs across a barrier if there is a driving force, such as a concentration gradient, or for charged molecules, a potential gradient, and if the barrier is permeable. This applies to almost all substances crossing the endothelial barrier **450** (FIG. **4B**) lining the peritubular capillaries **418** (FIG. **4B**). It applies to substances taking the paracellular route **460** (FIG. **4B**) around the tubular epithelium **452** (FIG. **4B**) and to some substances taking the transcellular route **466** (FIG. **4B**). Substances that are lipid solute, such as the blood gases or steroids, can diffuse directly through the lipid bilayer.

Most substances that are biologically important cannot penetrate lipid membranes. To cross a membrane, they need to move through specific integral membrane proteins, which are dividend into categories of channels **472** and transporters **474**. Channels **472** are small pores that permit, depending on their structure, water or specific solutes to diffuse through them. Examples of specific channels **472** include sodium channels and potassium channels that permit diffusion of these molecular species. Movement through channels **472** is passive (i.e., no external energy is required). The energy to drive the diffusion is inherent in the concentration gradient or, more specifically, the electrochemical gradient, because ions are driven through channels and around cells via the paracellular route **460** not only by gradients of concentration but also by gradients of voltage. Channels **472** represent a mechanism for rapidly moving across membranes large amounts of substances, which would otherwise diffuse slowly or not at all. The amount of material passing through an ion channel **472** can be controlled by opening and closing the channel pore.

Transporters **474**, like channels **472**, permit the transmembrane flux of a solute that is otherwise impermeable in the lipid bilayer. However, unlike channels **472**, many transporters **474** are extremely specific, transporting only 1 or at most a small class of substances. The specificity is usually coupled to a lower rate of transport because the transported solutes bind much more strongly to the transport protein. Furthermore, the protein must undergo a more elaborate cycle of conformational change to move the solute from one side of the membrane to the other.

Transporters **474** can be grouped into categories including uniporters **476**, symporters **478** and antiporters **479**, and primary active transporters (ATP) **480** according to basic functional properties. Uniporters **476** permit movement of a single solute species through the membrane. Movement through a uniporters **476** is like diffusion in that it is driven by concentration gradients, but is different in that the transported material moves through the uniporter protein rather than the membrane. Symporters **478** and antiporters **479** move two or more solute species in the same direction across a membrane (symporters) or in opposite directions across a membrane (antiporters). With symporters **478** and antiporters **479**, at least one of the solutes moves down its electrochemical gradient and provides the energy to move one or more of the other solutes up its electrochemical gradient. Primary active transporters **480** are membrane proteins that are capable of moving one or more solutes up their electrochemical gradients, using the energy obtained from the hydrolysis of adenosine triphosphate (ATP). Among the key primary active transporters in the kidney **104** (FIG. 1) is Na-K-ATPase (often referred to as the "sodium pump"), some form of which is present in all cells of the body. This transporter simultaneously moves sodium against its electrochemical gradient out of a cell and potassium against its gradient into a cell.

In light of the above-discussed systems **100** (FIGS. **1**, **2**, **3A**) and further in light of the above-discussed kidney structures, including the glomerulus **356**, the Bowman's capsule **358**, the tubule **354**, the peritubular capillary network **418**, the collecting duct , the afferent arteriole **360**, the efferent arteriole **362**, or the renal granular cell **361**, some beliefs of how the electrical energy stimulation may be targeted toward renal function modulation, and thus renal solute control, renal water control, and renal system blood pressure (i.e., examples of renal function-associated parameters) are discussed below.

### Electrical Energy Stimulation Targeted Toward Renal Solute Control:

Electrical modulation of glomerular filtrate solute control can be targeted toward the filtration by the glomerulus **356** (FIG. **3B**). Alternatively or additionally, concentration of a particular solute may be modulated via imparting electrical energy stimulation across various channels **472** (FIG. **4C**) (e.g., sodium channel, potassium channel) or transporters **474** (FIG. **4C**) (e.g., uniporter **476**, symporter **478** and antiporter **47**) with the tubule **354** (FIG. **4A**) or collecting duct.

As discussed above, movement through channels **472** is passive as the diffusion therethrough is due, in part, to specific solute concentration gradients, and more specifically, to the electrochemical gradient. Ions are driven through and around channels **472** not only by gradients due to the specific solute concentration, but also by gradients of voltage across the channel **472**. This sensitivity of channels **472** to voltage provides a mechanism to support the belief that channels **472** may be modulated via applied electrical energy stimulation. Regarding transporters **474**, it has been shown, such as in Blank, M. and Soo, L., Threshold for inhibition of Na, K-ATPase by ELF alternating currents, Bioelectromagnetics, Vol. 13, Issue 4 (Published Online Oct. 2005): 329-333, that alternating current can increase or decrease the ATP-splitting activity of the membrane enzyme Na-K-ATPase.

As further discussed above, maintenance of proper blood electrolyte (e.g., sodium, chlorine, or potassium) levels is a key function of the kidney. Supporting the premise that modulation of ion channels within the nephrons is possible includes studies, such as Teissie, J. and Tsong, T., Voltage Modulation of Na+/K+ Transport in Human Erythrocytes, Journal of Physiology (Paris), (May 1981); 77(9): 1043-1053 PMID: 6286955; Serpersu, E. H. and Tsong, T. Y., Activation of electrogenic Rb+ transport of (Na K)-A TPase by an electric field, J. Biol. Chem., (June 10, 1984); 259(11): 7155-62; Liu, D. S., Astumian, R. D., and Tsong, T. Y., Activation of Na+ and K+ pumping modes of (Na, K)-ATPase by an oscillating electric field, J. Biol. Chem., (May 5, 1990); 265(13): 7260-7 PMID: 2158997; and Serpersu, E. H., Tsong, T. Y., Stimulation of a ouabain-sensitive Rb+ uptake in human erthrocytes with an external electric field, J. Membr. Biol., (1983); 74(3): 191-201 PMID: 6887232, noting that modulation of sodium and potassium ion channels in human erythrocytes (red blood cells) via electrical energy stimulation has been accomplished. Further, ion channels in human erythrocytes can be selectively targeted by altering the frequency of the applied electrical energy stimulation. Specifically, sodium has been found to be sensitive to frequencies from 1 KHz to 100 KHz and potassium channels have been found to be sensitive to frequencies of about 1 MHz. *(See* Serpersu, E. H. and Tsong, T. Y., Activation of electrogenic Rb+ transport of (Na K)-ATPase by an electric field and Liu, D. S., Astumian, R. D., and Tsong, T. Y., Activation of Na+ and K+ pumping modes of (Na, K)-A TPase by an oscillating electric field, J. Biol. Chem.. The electric fields required to produce these effects are on the order of 10 V/cm. The half life of an ion channel opening due to applied electric fields is about 10 seconds (*see* Serpersu, E. H. and Tsong, T. Y., Activation of electrogenic Rb+ transport of (Na K)-ATPase by an electric field); thus, continuous application of electrical energy stimulation may not be required.

In addition to transcellular **466** (FIG. **4B**) routes, solute movement may also occur via paracellular **460** (FIG. **4B**) routes. It has been found that both solute concentrations and electric fields **306** (FIG. **3A**) play a role in paracellular solute movement. Solutes that can move via paracellular routes include urea, potassium, chloride, calcium, and magnesium. Paracellular **460** route sensitivity to electric fields **306** may allow modulation of these routes via imposition of electrical energy stimulation.

In addition to the work noted above with human erythrocytes, work, such as Burkhoff, D., Shemer, I., Felzen, B., Shimizu, J., Mika, Y., Dickstein, M., Prutchi, D., Darvish, N., Ben-Haim, S. A., Electric currents applied during the refractory period can modulate cardiac contractility in vitro and in vivo, Heart Failure Rev., (Jan. 2001); 6(1): 27-34 PMID: 11248765, has been conducted with cardiac contractility modulation via application of electric current during cardiac refractory periods. Application of electric current has been shown to modify calcium movement across cellular membranes during certain phases of cardiac myocyte action potential.

Glomerular filtration is dependent on solute size, hydrostatic and oncotic pressures and the electrical charge of individual solutes. For any given size, negatively charged macromolecules are filtered to a lesser extent, and positively charged macromolecules to a greater extent, then neutral molecules. The filtrate dependence on the solute's electrical charge is due to fixed negative charge within certain portions of the glomerular membrane. It is important to note that charge dependent filtration pertains only to macromolecules (e.g., albumin) and not mineral ions or low weight molecules (e.g., chloride or bicarbonate ions). It has been shown, such as in Kverneland, A., Feldt-Rasmussen, B., Vidal, P., Welinder, B., Bent-Hansen, L., Soegaard, U., and Decker, T., Evidence of changes in renal charge selectivity in patients with type 1 (insulin-dependent) diabetes mellitus, Diabetologia, (Sept. 1986): (9) 634-9, that alterations in the glomerular membrane charge influences filtration of albumin resulting in albuminuria. Thus, it may be possible to alter glomerular filtration of certain charged macromolecules by imposing electric fields **306** across the glomerulus **356**.

### Electrical Energy Stimulation Targeted Toward Renal Water Control:

Approximately 99% of the water in the glomerular filtrate is reabsorbed by the kidneys **104** (FIG. **1**). Reduction of the reabsorbed **414** (FIG. **4A**) portion of water within the nephrons **350** (FIG. **3B**) via application of electrical energy stimulation provides an opportunity to promote diuresis. Like conventional pharmaceutical diuretics, diuresis via imposition of electrical energy stimulation could be caused by increased excretion of sodium, which as noted above, may be manipulated via application of electrical energy stimulation.

Another potential method to promote diuresis is the application of electrical energy stimulation in a manner that modulates the peritubular capillary's **418** (FIG. **4A**) aquaporin sensitivity to antidiuretic hormone (ADH). Reducing the kidneys' **104** sensitivity to ADH will promote diuresis. ADH is secreted by the posterior pituitary and acts on the peritubular capillary of the kidneys **104** to cause them to reabsorb water, thereby concentrating the urine. Since it is believed that most aquaporins are virtually impermeable to ions, control of aquaporin function via application of electrical energy stimulation may be difficult. If aquaporin function is insensitive to applied electrical energy stimulation, it would be advantageous in one regard since it prevents unintentional change in aquaporin function when the electrical energy stimulation is targeted at other renal structures.

### Electrical Energy Stimulation Targeted Toward Systemic Blood Pressure:

Renal control of blood pressure results from both the regulation of blood volume within the vascular tree via control of sodium and water (e.g., using the techniques discussed above) and by the excretion of chemical agents, such as rein and angiotension II, that alter vascular resistances to correct blood pressure. Renin, for example, is released by renal granular cells **361** (FIG. **3B**). It is believed that the release of renin by the renal granular cells **361** may be accomplished via application of electrical energy stimulation.

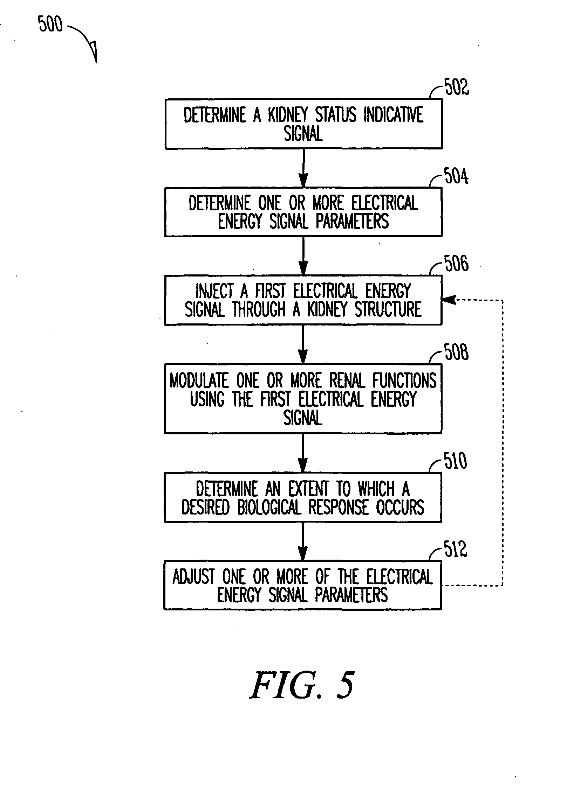

FIG. **5** illustrates a method **500** of modulating one or more renal functions by applying electrical energy stimulation to one or more kidney structures (e.g., a glomerulus, a Bowman's capsule, a macula densa, a tubule, a peritubular capillary network, a collecting duct, an afferent arteriole, an efferent arteriole, or a renal granular cell). At **502**, a kidney status indicative signal is determined. Determination can be from, for example, an internal sensor module **226** (FIG. **2**), an implantable sensor **227** (FIG. **2**) or information communicated to the IMD **110** (FIG. **2**) via an external user interface **118** (FIG. **2**). In certain examples, the kidney status indicative signal includes information about one or more renal function-associated parameters, such as whether a then-current value of the one or more parameters is associated with a current or impending disease state. If it is determined that one or more renal function-associated parameters values are indicative of disease, one or more electrical energy signal parameters aimed at normalizing the parameters are determined at **504**. In various examples, the one or more electrical energy signal parameters include an energy injection location, an energy injection duration, an energy injection intensity, an energy injection frequency, an energy injection polarity, an energy injection electrode configuration, or an energy injection waveform. In certain examples, the electrical energy signal includes a pulsed voltage signal with approximately a zero average amplitude, a frequency between approximately 1KHz and approximately 1MHz, and a peak-to-peak amplitude sufficient to produce an electric field strength of approximately 10 volts per centimeter.

At **506**, a first electrical energy signal characterized by the one or more electrical energy stimulation parameters is internally injected between a first and a second electrode, such that a substantially large portion of the signal flows through a subject's kidney(s), and more specifically, at least one of the glomerulus, the Bowman's capsule, the macula densa, the tubule, the peritubular capillary network, the collecting duct, the afferent arteriole, the efferent arteriole, or the renal granular cell. At **508**, one or more renal functions are modulated using the first electrical energy signal. In various examples, modulation of the one or more renal functions includes affecting a change of the one or more renal function-associated parameters (e.g., an electrolyte level, a water level, a metabolic waste level, a pharmacological agent level, a hormone level, a blood pressure level, an erythropoietin level, a vitamin D level, a glucose level, a pH level, or a glomerulus filtration rate level).

At **510**, an extent to which the desired biological response of the one or more renal function-associated parameters occurs is determined. In certain examples, this can include a re-determination of the kidney status indicative signal and comparison of such signal with stored desired parameter values. At **512**, one or more of the electrical energy signal parameters can optionally be adjusted in light of the extent determined at **510**. The process can subsequently return to **506** for further electrical energy stimulation.

Renal function modulation via application of electrical energy stimulation is discussed herein. The electrical energy stimulation can be used to supplement or in lieu of existing renal failure treatments (e.g., drug therapy, hemodialysis, or transplantation) to keep kidney subjects in a state of relative well-being by preventing, delaying, or minimizing renal conditions including, for example, chronic kidney disease and end stage renal failure. It is believed that by selectively manipulating one or more kidney structures that one or more renal functions may be modulated in a desired way, such as the way non-disease state kidneys would normally function. By modulating the one or more renal functions, a desired biological response of one or more renal function-associated parameters may be effectuated, thereby treating or preventing associated diseases (e.g., hypertension, edema, heart failure, blood electrolyte imbalances, and others).

It is to be understood that the above description is intended to be illustrative, and not restrictive. For instance, while a majority of the foregoing discusses electrical energy stimulation in the form of an electric current or an associated electric field, the present subject matter may also include other forms of electrical energy stimulation, such as magnetic fields or magnetic flux to modulate one or more renal functions. For instance, according to at least one study, such as is found in Blank, M. and Soo L., Frequency Dependence of NA,K-ATPase Function in Magnetic Fields, Bioelectrochemistry and Bioenergetics, May 1997: 42(2) 231-234, Na-K-ATPase function has been found to be dependent on magnetic energy.

Although specific embodiments have been illustrated and described herein, it will be appreciated by those of ordinary skill in the art that any arrangement which is calculated to achieve the same purpose may be substituted for the specific embodiment shown. This patent document is intended to cover adaptations or variations of the present subject matter. It is to be understood that the above description is intended to be illustrative, and not restrictive. Combinations of the above embodiments and other embodiments will be apparent to those of skill in the art upon reviewing the above description. The scope of the present subject matter should be determined with reference to the appended claims, along with the full scope of equivalents to which such claims are entitled.

## Claims

1. A system for applying a stimulus to at least one of a glomerulus, a Bowman's capsule, a macula densa, a tubule, a peritubular capillary network, a collecting duct, an afferent arteriole, an efferent arteriole, or a renal granular cell within a kidney (104) of a subject (102), the system comprising:
a first electrode (208) being configured for disposition within the subject and proximal to the kidney;
a second electrode (212, 214) being located on a housing (210) or a header (206) of an implantable medical device (110);
an electrical energy delivery circuit (220) coupled to the first electrode and the second electrode, the electrical energy delivery circuit disposed, at least in part, within the implantable medical device (110), the electrical energy delivery circuit configured to generate a first electrical energy signal;
wherein the first electrode and the second electrode are configured to direct a substantially large portion of the first electrical energy signal through at least one of the glomerulus, the Bowman's capsule, the macula densa, the tubule, the peritubular capillary network, the collecting duct, the afferent arteriole, the efferent arteriole, or the renal granular cell;
wherein the first electrical energy signal includes a pulsed voltage having a peak-to-peak amplitude to produce an electric field strength of approximately 10 volts per centimeter, and is configured to modulate activity of adenosine triphosphate (ATP) to control one or more renal functions;
**characterized in that** the first electrical energy signal has a frequency between about 1 KHz and about 1 MHz.

2. The system of claim 1, further comprising a measurement unit configured to measure one or more parameters associated with the one or more renal functions.

3. The system of claim 2, wherein the one or more measured parameters associated with the one or more renal functions include one or more of an electrolyte level, a water level, a metabolic waste level, a pharmacological agent level, a hormone level, a blood pressure level, an erythropoietin level, a vitamin D level, a glucose level, a pH level, or a glomerulus filtration rate level.

4. The system of claims 2 or 3, further comprising a processor coupled with the electrical energy delivery circuit, the processor configured to control the electrical energy delivery circuit using information about the one or more parameters associated with the one or more renal functions.

5. The system of claim 4, wherein the control of the electrical energy delivery circuit includes control of one or more of an energy injection location, an energy injection duration, an energy injection intensity, an energy injection frequency, an energy injection polarity, an energy injection electrode configuration, or an energy injection waveform of the first electrical energy signal using information about the one or more parameters associated with the one or more renal functions.

6. The system of claims 4 or 5, further comprising an external user interface unit communicatively coupled to the processor, the external user interface unit configured to at least one of display information about the one or more parameters associated with the one or more renal functions, provide an input of the subject's health related information, or allow external control of the electrical energy signal.

7. The system of any of claims 1-6, wherein the first electrode is disposed on a renal vasculature insertable lead.

8. The system of any of claims 1-6, wherein the first electrode is disposed on a urethra insertable lead.

9. The system of any of claims 1-8, wherein the electrical energy delivery circuit is configured to inject an electric current between the first electrode and the second electrode.

10. The system of any of claims 1-9, wherein the implantable medical device includes a cardiac therapy unit, the cardiac therapy unit configured to deliver at least one of a bradycardia therapy, a tachycardia therapy, or a cardiac resynchronization therapy to the subject.

11. The system of any of claims 1-10, wherein the first electrical energy signal includes a pulsed voltage signal having approximately a zero average amplitude.

## Patentansprüche

1. System zum Ausüben eines Stimulus auf zumindest eine/einen von einem Glomerulus, einer Bowman-Kapsel, einer Makula Densa, einem Tubulus, einem peritubulösen Kapillarnetzwerk, einem Sammelleiter, einer zuführenden Arteriole, einer ableitenden Arteriole oder einer Nierengranularzelle innerhalb einer Niere (104) eines Subjekts (102), welches System aufweist:
eine erste Elektrode (208), die zur Anordnung innerhalb des Subjekts und proximal von der Niere ausgebildet ist;
eine zweite Elektrode (212, 214), die sich auf einem Gehäuse (210) oder einem Aufsatz (206) einer implantierbaren medizinischen Vorrichtung (110) befindet;
eine Zuführungsschaltung (220) für elektrische Energie, die mit der ersten Elektrode und der zweiten Elektrode gekoppelt ist, wobei sich die Zuführungsschaltung für elektrische Energie zumindest teilweise innerhalb der implantierbaren medizinischen Vorrichtung (110) befindet und wobei die Zuführungsschaltung für elektrische Energie ausgebildet ist zum Erzeugen eines ersten elektrischen Energiesignals;
wobei die erste Elektrode und die zweite Elektrode ausgebildet sind zum Richten eines im Wesentlichen großen Teils des ersten elektrischen Energiesignals durch zumindest eine/einen von dem Glomerulus, der Bowman-Kapsel, der Makula Densa, dem Tubulus,
dem peritubulösen Kapillarnetzwerk, dem Sammelleiter, der zuführenden Arteriole, der ableitenden Arteriole oder der Nierengranularzelle;
wobei das elektrische Energiesignal eine gepulste Spannung mit einer Spitze-zu-Spitze-Amplitude enthält, um eine elektrische Feldstärke von angenähert 10 Volt pro Zentimeter zu erzeugen, und ausgebildet ist zum Modulieren der Aktivität von Adenosintriphosphat (ATP), um eine oder mehr Nierenfunktionen zu steuern;
**dadurch gekennzeichnet, dass** das erste elektrische Energiesignal eine Frequenz zwischen etwa 1 KHz und etwa 1 MHz hat.

2. System nach Anspruch 1, weiterhin aufweisend eine Messeinheit, die ausgebildet ist zum Messen von einem oder mehr Parametern, die mit der einen oder mehr Nierenfunktionen assoziiert sind.

3. System nach Anspruch 2, bei dem der eine oder mehr gemessene Parameter, die mit der einen oder mehr Nierenfunktionen assoziiert sind, einen oder mehr von einem Elektrolytpegel, einem Wasserpegel, einem metabolischen Reststoffpegel, einem Pegel für pharmakologisches Mittel, einem Hormonpegel, einem Blutdruckpegel, einem Erythropoietinpegel, einem Vitamin-D-Pegel, einem Glukosepegel, einem pH-Pegel oder einem Pegel für eine Glomerulusfiltrierungsrate enthalten.

4. System nach Anspruch 2 oder 3, weiterhin aufweisend einen mit der Zuführungsschaltung für elektrische Energie gekoppelten Prozessor, welcher Prozessor ausgebildet ist zum Steuern der Zuführungsschaltung für elektrische Energie unter Verwendung von Informationen über den einen oder mehr Parameter, die mit der einen oder mehr Nierenfunktionen assoziiert sind.

5. System nach Anspruch 4, bei dem die Steuerung der Zuführungsschaltung für elektrische Energie die Steuerung von einem oder mehr eines Energieinjektionsortes, einer Energieinjektionsdauer, einer Energieinjektionsintensität, einer Energieinjektionsfrequenz, einer Energieinjektionspolarität, einer Energieinjektions-Elektrodenkonfiguration oder einer Energieinjektions-Wellenform des ersten elektrischen Energiesignals unter Verwendung von Informationen über den einen oder mehr Parameter, die mit der einen oder mehr Nierenfunktionen assoziiert sind, enthält.

6. System nach Anspruch 4 oder 5, weiterhin aufweisend eine externe Benutzerschnittstelleneinheit, die kommunikativ mit dem Prozessor gekoppelt ist, welche externe Benutzerschnittstelleneinheit ausgebildet ist zum zumindest einen von Anzeigen von Informationen über den einen oder mehr Parameter, die mit der einen oder mehr Nierenfunktionen assoziiert sind, Vorsehen einer Eingabe von gesundheitsbezogenen Informationen des Subjekts oder Ermöglichen einer externen Steuerung des elektrischen Energiesignals.

7. System nach einem der Ansprüche 1- 6, bei dem die erste Elektrode auf einem in ein Nierengefäß einführbaren Leiter angeordnet ist.

8. System nach einem der Ansprüche 1 - 6, bei dem die erste Elektrode auf einem in die Harnröhre einführbaren Leiter angeordnet ist.

9. System nach einem der Ansprüche 1 - 8, bei dem die Zuführungsschaltung für elektrische Energie ausgebildet ist zum Injizieren eines elektrischen Stroms zwischen der ersten Elektrode und der zweiten Elektrode.

10. System nach einem der Ansprüche 1- 9, bei dem die implantierbare medizinische Vorrichtung eine Herztherapieeinheit enthält, welche Herztherapieeinheit ausgebildet ist zum Liefern zumindest einer von einer Bradycardietherapie, einer Tachycardietherapie oder einer Herzresynchronisationstherapie zu dem Subjekt.

11. System nach einem der Ansprüche 1 - 10, bei dem das erste elektrische Energiesignal ein gepulstes Spannungssignal mit einer Durchschnittsamplitude von angenähert null enthält.

## Revendications

1. Système pour appliquer un stimulus à au moins un élément pris parmi un glomérule rénal, une capsule de Bowman, une macula densa, un tubule, un réseau capillaire péritubulaire, un conduit de collecte, une artériole afférente, une artériole afférente et une cellule granulaire rénale à l'intérieur d'un foie (104) d'un sujet (102), le système comprenant :
une première électrode (208) qui est configurée de manière à pouvoir être disposée à l'intérieur du sujet et de façon proximale par rapport au foie ;
une seconde électrode (212, 214) qui est localisée sur un boîtier (210) ou une embase (206) d'un dispositif médical implantable (110) ;
un circuit de délivrance d'énergie électrique (220) couplé à la première électrode et à la seconde électrode, le circuit de délivrance d'énergie électrique étant disposé, au moins en partie, à l'intérieur du dispositif médical implantable (110), le circuit de délivrance d'énergie électrique étant configuré pour générer un premier signal d'énergie électrique, **caractérisé en ce que** le premier signal d'énergie électrique présente une fréquence entre environ 1 kHz et environ 1 MHz, dans lequel
la première électrode et la seconde électrode sont configurées pour diriger une partie substantiellement importante du premier signal d'énergie électrique au travers d'au moins un élément pris parmi le glomérule rénal, la capsule de Bowman, la macula densa, le tubule, le réseau capillaire péritubulaire, le conduit de collecte, l'artériole afférente, l'artériole afférente et la cellule granulaire rénale, dans lequel
le premier signal d'énergie électrique inclut une tension pulsée présentant une amplitude crête-à-crête pour produire une intensité de champ électrique d'approximativement 10 volts par centimètre et est configuré pour moduler l'activité de l'adénosine triphosphate (ATP) afin de contrôler une ou plusieurs fonction(s) rénale(s).

2. Système selon la revendication 1, comprenant en outre une unité de mesure configurée pour mesurer un ou plusieurs paramètre(s) associé(s) aux une ou plusieurs fonction(s) rénale(s).

3. Système selon la revendication 2, dans lequel les un ou plusieurs paramètre(s) mesuré(s) associé(s) aux une ou plusieurs fonction(s) rénale(s) inclu(en)t un ou plusieurs des niveaux qui suivent : un niveau d'électrolyte, un niveau d'eau, un niveau de rejet métabolique, un niveau d'agent pharmacologique, un niveau d'hormone, un niveau de pression sanguine, un niveau d'érythropoïétine, un niveau de vitamine D, un niveau de glucose, un niveau de pH et un niveau de taux de filtration de glomérule rénal.

4. Système selon la revendication 2 ou 3, comprenant en outre un processeur couplé au circuit de délivrance d'énergie électrique, le processeur étant configuré pour commander le circuit de délivrance d'énergie électrique en utilisant une information concernant les un ou plusieurs paramètre(s) associé(s) aux une ou plusieurs fonction(s) rénale(s).

5. Système selon la revendication 4, dans lequel la commande du circuit de délivrance d'énergie électrique inclut la commande d'un ou de plusieurs éléments pris parmi une localisation d'injection d'énergie, une durée d'injection d'énergie, une intensité d'injection d'énergie, une fréquence d'injection d'énergie, une polarité d'injection d'énergie, une configuration d'électrode(s) d'injection d'énergie et une forme d'onde d'injection d'énergie du premier signal d'énergie électrique en utilisant une information concernant les un ou plusieurs paramètre(s) associé(s) aux une ou plusieurs fonction(s) rénale(s).

6. Système selon la revendication 4 ou 5, comprenant en outre une unité d'interface utilisateur externe couplée en communication au processeur, l'unité d'interface utilisateur externe étant configurée pour réaliser au moins une action prise parmi l'affichage d'une information concernant les un ou plusieurs paramètre(s) associé(s) aux une ou plusieurs fonction(s) rénale(s), l'application d'une entrée de l'information rapportée à la santé du sujet et la possibilité de la commande externe du signal d'énergie électrique.

7. Système selon l'une quelconque des revendications 1-6, dans lequel la première électrode est disposée sur une connexion insérable de vasculature rénale.

8. Système selon l'une quelconque des revendications 1-6, dans lequel la première électrode est disposée sur une connexion insérable d'urètre.

9. Système selon l'une quelconque des revendications 1-8, dans lequel le circuit de délivrance d'énergie électrique est configuré pour injecter un courant électrique entre la première électrode et la seconde électrode.

10. Système selon l'une quelconque des revendications 1-9, dans lequel le dispositif médical implantable inclut une unité de thérapie cardiaque, l'unité de thérapie cardiaque étant configurée pour délivrer au moins une thérapie prise parmi une thérapie de bradycardie, une thérapie de tachycardie et une thérapie de resynchronisation cardiaque au sujet.

11. Système selon l'une quelconque des revendications 1-10, dans lequel le premier signal d'énergie électrique inclut un signal de tension pulsé présentant approximativement une amplitude moyenne de zéro.
